# FASCICULE DE BREVET EUROPEEN

(11) **EP 0 734 513 B1**
(45) Date de publication et mention de la délivrance du brevet: **29.07.1998**
(21) Numéro de dépôt: 95903832.4
(22) Date de dépôt: 14.12.1994
(51) Int. Cl.: G01N 1/24, G01N 33/00

(54) **PROCEDE ET DISPOSITIF D'ANALYSE RAPIDE EN CONTINU DE MELANGE GAZEUX**
VERFAHREN UND VORRICHTUNG ZUR SCHNELLEN KONTINUIERLICHEN ANALYSE EINES GASGEMISCHES
FAST CONTINUOUS GAS MIXTURE ANALYSIS METHOD AND DEVICE

(30) Priorité: 14.12.1993 FR 9315197
(43) Date de publication de la demande: 02.10.1996
(73) Titulaire: USINOR, 92800 Puteaux (FR)
(72) Inventeur: GAGGIOLI, André, F-57210 Maizières-lès-Metz (FR); FIORELLI, Sylvain, F-57000 Metz (FR)
(74) Mandataire: Ventavoli, Roger
(86) Numéro de dépôt international: FR9401462
(87) Numéro de publication internationale: WO9516904

(56) Documents cités:
- EP-A- 0 095 802
- EP-A- 0 208 438
- DE-A- 2 301 264
- DE-A- 3 339 073
- US-A- 4 484 481

## Description

L'invention concerne l'analyse en continu de mélanges gazeux en particulier de gaz chargés en poussières ou impuretés de toutes sortes, tels que des fumées, notamment des fumées d'aciéries, d'incinérateurs, de chaînes d'agglomérations, ...etc.

L'analyse de gaz est classiquement réalisée par des analyseurs qui permettent de déterminer la teneur d'un ou plusieurs gaz composants un mélange gazeux. Par exemple, dans des installations d'aciérie électrique comprenant un four à arc, on utilise classiquement un analyseur de gaz pour déterminer la teneur résiduelle en oxygène, après combustion, des fumées aspirées dans le dispositif de captage dont est pourvu le four, afin de pouvoir contrôler le processus de combustion.

Un tel analyseur est par exemple un analyseur au zircone, tel que celui commercialisé par la société "Servomex" sous la référence "Série 700 B". Cet analyseur comprend une tête de sonde qui comporte une cellule au zircone de mesure d'oxygène. La tête de sonde est montée directement sur le conduit de transport des fumées à analyser. L'échantillon gazeux est prélevé dans ce conduit par un tube de prélèvement, passe à travers un filtre placé dans la tête de sonde, par exemple un filtre en acier inoxydable poral, puis dans la cellule au zircone. Le prélèvement est effectué par aspiration au moyen d'un dispositif d'aspiration, placé en aval de la cellule au zircone.

D'autres types d'analyseurs de gaz sont également utilisés pour analyser divers constituants gazeux, tels que des analyseurs à infrarouge, à conductibilité thermique, paramagnétique, spectromètre de masse, hygromètre, ...etc.

Dans de nombreux cas, et notamment dans le cas de l'analyse des fumées de combustion de réacteurs métallurgiques, comme le four à arc par exemple, il est primordial d'obtenir une analyse rapide afin de pouvoir intervenir immédiatement sur les paramètres de réglage de la combustion au besoin. Or, les dispositifs d'analyse, même les plus performants, ont nécessairement un temps de réponse relativement long dû à leur ligne de prélèvement. Cette ligne, qui va du piquage sur la conduite d'évacuation des fumées jusqu'à l'entrée de l'analyseur, définit un "volume mort" correspondant à la série de traitements de nettoyage et de préparation que doivent subir les fumées prélevées préalablement à leur analyse. C'est la durée que met le courant gazeux pour parcourir ce volume mort qui est le facteur souvent déterminant du temps de réponse de l'analyse. Or, en analyse continue de fumées chargées en poussières, on a constaté que le temps de réponse de l'analyse était notamment fonction du degré de colmatage du filtre placé dans la tête de sonde.

Les fumées émises par un four à arc, contiennent classiquement une quantité de poussières qui atteint 30 g/Nm³ de gaz, avec une granulométrie inférieure à 100 µm. Dans ces conditions, le filtre de l'analyseur se colmate rapidement, et le temps de réponse de l'analyse augmente à mesure que le filtre se colmate. Des essais réalisés sur le captage d'un four à arc ont révélé que le filtre pouvait se colmater après 24 heures de fonctionnement du four et nécessitait son remplacement. L'échange du filtre nécessite un arrêt prolongé de l'analyseur. Cette opération nécessitant une intervention manuelle, il faut attendre le refroidissement de la tête de sonde qui est thermostatée à 840°C en fonctionnement normal avant de pouvoir changer le filtre, contrôler l'étanchéité, remettre l'analyseur sous tension, attendre le retour de la tête de sonde à la température de régime, et réétalonner l'analyseur avant de le remettre en service. Outre le temps passé à cette intervention et le coût du filtre, l'arrêt de l'analyseur entraîne nécessairement une interruption de l'analyse, qui peut durer jusqu'à quatre heures. Pendant ce temps, les résultats de l'analyse ne peuvent plus être fournis au système de régulation du captage, ce qui est préjudiciable à l'exploitation du four et à la sécurité.

On connaît des dispositifs d'analyse à temps de réponse écourté basés sur un débit de prélèvement bien supérieur au débit d'analyse, afin d'assurer un balayage rapide du "volume mort" constitué par la ligne de prélèvement. L'un de ces dispositifs, décrit, par exemple, dans DE-A-3339073, semble cependant d'application limitée à des analyses séquentielles de courte durée, de sorte que le colmatage progressif du filtre ne constitue pas une gêne réelle. Un autre, décrit dans EP-A-O 208 438, permet, lui, d'assurer une analyse en continu, mais moyennant la mise en oeuvre de deux lignes de prélèvement en parallèle, fonctionnant en alternance.

L'invention a pour but de résoudre les difficultés précitées et notamment d'éviter que les colmatages fréquents du filtre de l'analyseur conduisent à des arrêts prolongés de l'analyse, de prolonger au maximum la durée entre deux interventions d'entretien du dispositif d'analyse, et de garantir pendant toute cette période, sans discontinuité, un temps de réponse minimal de l'analyse.

Avec ces objectifs en vue, l'invention a pour objet un procédé d'analyse rapide en continu d'un mélange gazeux, selon lequel on fait passer dans un analyseur de gaz un échantillon prélevé dans ledit mélange gazeux, on utilise des moyens de filtration, placés en aval d'une sonde de prélèvement et en amont d'une pompe, pour épurer le mélange prélevé, on prélève ledit mélange à travers lesdits moyens de filtration, avec un débit élevé supérieur à un débit d'entrée voulu dans l'analyseur, et on dirige le mélange gazeux vers l'analyseur sous ledit débit d'entrée voulu, le débit restant constituant un débit de fuite que l'on évacue sans le faire passer dans l'analyseur, caractérisé en ce qu'on régule le débit dans l'analyseur, on mesure le débit de fuite évacué, et on procède à un nettoyage des moyens de filtration par un balayage à contre courant desdits moyens de filtration avec un gaz propre, lorsque le débit défini mesuré atteint un seuil minimal prédéterminé.

Comme on l'aura compris, l'idée à la base de l'invention a été d'avoir associé un débit de prélèvement filtré nettement supérieur au débit d'analyse, à un nettoyage automatique des filtres par balayage à contre courant lorsque le débit de fuite, utilisé comme moyen de réglage du débit d'analyse à une valeur constante, montre que le débit de prélèvement s'approche de trop près du débit d'analyse en raison du colmatage progressif des filtres au cours du processus d'analyse en continu.

Comme on l'a dit, le temps de réponse de l'analyse dépend notamment du temps mis par l'échantillon prélevé dans le conduit de transport des fumées pour parvenir à la cellule d'analyse de l'analyseur. Pour une ligne de prélèvement donnée (volume mort donné), ce temps de réponse dépend donc du débit du mélange gazeux prélevé. Toutes autres caractéristiques du circuit étant constantes, plus le débit sera élevé, plus l'échantillon prélevé parvient rapidement à l'analyseur. Or, le débit traversant l'analyseur est naturellement limité par la constitution même de l'analyseur.

Selon l'invention, en dimensionnant les éléments du circuit en amont de la pompe, et la pompe elle-même, pour assurer un débit plus élevé que celui pouvant transiter dans l'analyseur, et en évacuant à la sortie de la pompe le débit excédentaire, on réduit ainsi le temps de transfert des gaz prélevés, tout en délivrant à l'analyseur le débit relativement faible dont il a besoin.

De plus, le filtre d'analyseur ne se colmatant plus, ou beaucoup moins rapidement, en raison de la filtration faite en amont sur le débit de prélèvement, le débit d'analyse peut être maintenu en permanence au-dessus d'un seuil minimal prédéfini, seul le débit excédentaire (débit de fuite) étant susceptible de diminuer lorsque les moyens de filtration du débit de prélèvement se colmatent progressivement.

En toute rigueur, on comprend que le débit de fuite peut tendre vers zéro sans préjudicier à la validité de l'analyse. Mais dans ce cas, le débit d'analyse serait égal au débit de prélèvement et la conséquence serait un temps de réponse d'analyse élevé, voire excessif pour une maîtrise correcte du processus industriel en cours nécessitant cette analyse.

En fait, le temps de réponse est le plus court au début, sur filtres propres et s'allonge à mesure que l'on est amené à réduire le débit de fuite pour maintenir constant le débit d'analyse.

Conformément à une mise en oeuve avantageuse de l'invention, on déclenchera la procédure de nettoyage des filtres, par balayage à contre courant, bien avant que le débit de fuite s'approche de zéro, le seuil de déclenchement étant à déterminer en fonction du temps de réponse maximal que l'on peut accepter pour l'utilisation industrielle en cours.

Pour fixer les idées, dans le cas d'un four à arc de 120 tonnes, on a visé un temps de réponse maximal de 15 secondes. Le volume mort étant en l'espèce d'environ 1,5 l et l'analyseur fonctionnant sous un débit nominal de 200 I/h à maintenir constant, on a obtenu un temps de réponse de 7 secondes environ en ligne propre tirant à 800 I/h (le bébit de fuite étant alors réglé à 600 I/h). A mesure que les filtres se sont colmatés, le temps de réponse a augmenté jusqu'à 11 secondes lorsque le débit de prélèvement a chuté à 600 I/h, le débit de fuite étant alors réglé à 400 I/h. On a choisi cette valeur de 400 l/h comme seuil de déclenchement du contre courant de nettoyage, car on ne souhaitait pas trop excéder 10 secondes au temps de réponse pour l'analyse.

Selon une disposition particulière, pour l'application du procédé à l'analyse rapide des fumées de captage d'un four à arc, on dimensionne la ligne de prélèvement (donc le volume mort) de manière à pouvoir procéder au nettoyage des moyens de filtration et à un réétalonnage de l'analyseur seulement quand le four est mis hors tension ou que sa voûte est ouverte. Ainsi, en commandant systématiquement le nettoyage des moyens de filtration lorsque l'alimentation électrique du four est arrêtée (ou que sa voûte est ouverte), on peut éviter d'avoir besoin de nettoyer les moyens de filtration au cours des phases d'élaboration de l'acier qui provoquent le plus de fumées chargées de poussières mais qui nécessitent en continu l'analyse rapide de l'oxygène résiduel contenu dans les fumées après combustion pour la régulation du captage de fumées. On assure donc une analyse correcte rapide et continue pendant toute la durée de ces phases, l'analyse n'étant alors interrompue que pendant les phases où la régulation du captage peut être supprimée sans risques.

L'invention a aussi pour objet un dispositif d'analyse rapide en continu d'un mélange gazeux comprenant un analyseur de gaz, une ligne de prélèvement comportant une sonde de prélèvement, une pompe pour faire passer dans l'analyseur un échantillon du mélange gazeux prélevé par la sonde, et des moyens de filtration, placés en aval de la sonde, la pompe étant placée en aval des moyens de filtration, et la sortie de la pompe étant reliée, d'une part, à l'analyseur par un conduit de liaison, et, d'autre part, à une évacuation par un conduit d'évacuation , dispositif caractérisé en ce qu'il comporte un régulateur de débit, placé en aval de la pompe, pour assurer un débit de mélange gazeux sensiblement constant dans le conduit de liaison, des moyens d'injection d'un gaz propre raccordés en aval des moyens de filtration, pour faire circuler le dit gaz propre à contre courant dans les moyens de filtration, des moyens de mesure du débit dans le conduit d'évacuation, et des moyens de commande pour commander l'injection de gaz propre quand ledit débit dans le conduit d'évacuation atteint un seuil minimal prédéterminé.

Préférentiellement, les moyens de filtration comprennent un filtre primaire pour retenir les particules de dimensions supérieures à une granulométrie prédéterminée et un condenseur, placé en aval du filtre primaire. Le filtre primaire permet d'éliminer la majeure partie des poussières des fumées prélevées, et le condenseur sert à refroidir les fumées préfiltrées, pour condenser la vapeur d'eau qu'elles contiennent et retenir les vapeurs et aérosols acides, ainsi que les fines particules de poussière qui on pu traverser le filtre primaire.

Selon une disposition complémentaire, le dispositif comporte des moyens d'isolement du circuit placés en amont des moyens de filtration pour isoler le circuit de la sonde, et des moyens pour injecter dans le circuit un gaz étalon. L'injection d'un gaz étalon, tel qu'un mélange comportant une teneur bien définie en gaz à analyser, permet de réétalonner régulièrement l'analyseur, par exemple après chaque nettoyage du filtre primaire.

Par ailleurs, en faisant circuler un tel gaz étalon, préférentiellement alors un gaz neutre tel que de l'azote, à partir des moyens de filtration, et moyennant d'avoir préalablement purgé tout le circuit avec le même gaz, en mettant l'analyseur en service, on peut contrôler l'étanchéité de la ligne de prélèvement en amont de la pompe, tout défaut d'étanchéité provoquant l'entrée du mélange gazeux ambiant dans le circuit, les composants de ce mélange autres que le gaz étalon étant détectés par l'analyseur.

D'autres caractéristiques et avantages apparaîtront dans la description qui va être faîte d'un dispositif, conforme à l'invention, d'analyse rapide en continu du taux d'oxygène contenu dans les fumées issues d'un four à arc d'aciérie, et de son procédé d'utilisation.

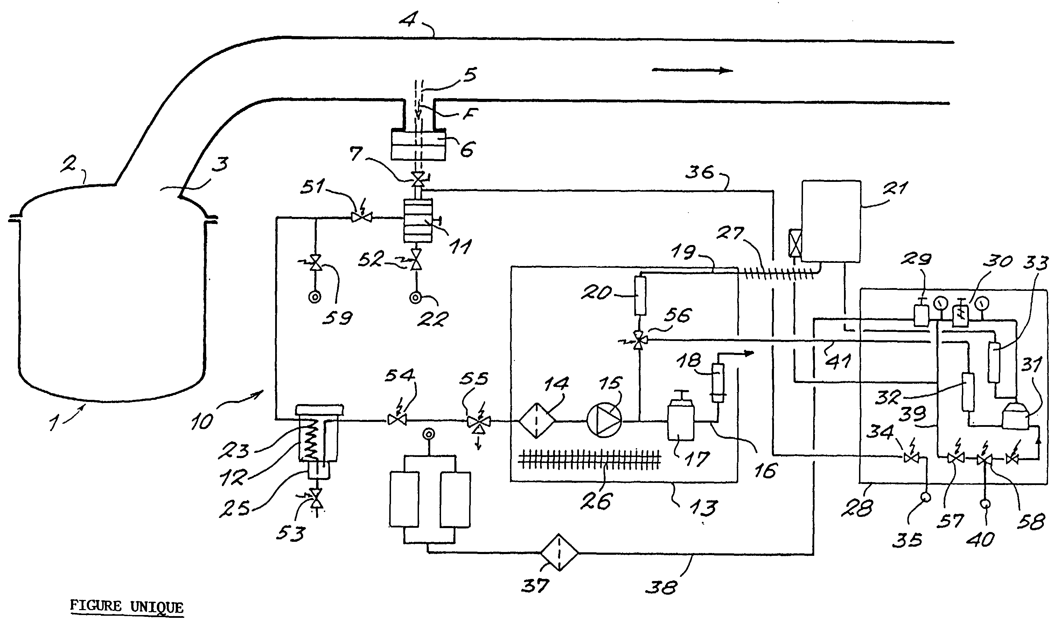

On se reportera aux dessins annexés dont l'unique figure représente le schéma du dispositif, installé sur le conduit de captage des fumées du four.

Le four à arc comprend une cuve 1 et une voûte 2 relevable percée d'un orifice 3 de sortie des fumées, sur lequel est raccordé un conduit 4 de captage et de transport des fumées pour les diriger vers un ensemble de filtration non représenté.

Le dispositif d'analyse comprend un analyseur d'oxygène 21 et une ligne de prélèvement comportant, reliés successivement dans le sens de circulation des fumées :
- une sonde de prélèvement 5, constituée d'un tube en acier fixé sur le conduit 4 par une bride 6, et qui pénètre dans le conduit,
- des moyens d'isolement de la sonde 5 du reste du circuit, par exemple une vanne d'isolement 7,
- des moyens de filtration 10 comprenant un filtre primaire 11 et un condenseur 12 en aval du filtre primaire,
- un coffret 13 contenant une cartouche filtrante jetable 14 et une pompe 15, dont la sortie est reliée d'une part à un conduit d'évacuation 16 sur lequel est placé un régulateur de débit 17 et un débitmètre à seuil 18, et d'autre part, à un conduit de liaison 19 comportant un second débitmètre 20,

L'analyseur d'oxygène 21 à électrolyte solide au zircone, est raccordé au conduit de liaison 19.

La vanne d'isolement 7 et le filtre primaire 11 sont placés immédiatement à la sortie de la sonde 5 pour éviter l'encrassement de la ligne de prélèvement.

Le filtre primaire 11 est de type connu, préférentiellement constitué d'un tube en céramique de porosité de l'ordre de 20 µm, logé dans un corps de filtre en acier inoxydable chauffé par exemple à environ 60°C pour éviter des condensations à l'intérieur du filtre. La porosité du tube est volontairement élevée pour éviter qu'il ne se colmate trop rapidement.

L'intérieur du tube est relié à la sonde 5. Le corps du filtre est relié par une électrovanne 52 à un circuit, représenté par le raccord 22, d'azote sous pression, par exemple à 7 bars, de manière que, lorsqu'on ouvre l'électrovanne 52, l'azote circule dans le filtre à contre courant du sens (flèche F) des fumées prélevées, pour balayer les poussières déposées à l'intérieur du filtre et le décolmater.

Le condenseur 12 comprend un serpentin 23 en cuivre chromé intérieurement, placé dans une cuve 24 et refroidi par une circulation d'eau. Les fumées qui passent dans le serpentin sont ainsi refroidies et la vapeur d'eau contenue dans ces fumées se condense et s'écoule dans le fond de la cuve 24 où elle est recueillie par un pot de récupération 25. En cours de fonctionnement, après quelques temps, le niveau des condensats dans le pot 25 est tel que les fumées sortant à la partie inférieur du serpentin peuvent barboter, ce qui permet de retenir dans le condensat les vapeurs et aérosols acides ainsi que les fines particules de poussière ayant traversé le filtre primaire. Le condenseur permet aussi de retenir environ 90 % des fines particules restant dans les fumées après le filtre primaire. Le pot de récupération est pourvu d'une électrovanne de vidange 53 qui permet de purger cycliquement le condenseur et d'évacuer les condensats.

La cartouche filtrante 14, placée en amont de la pompe, a un pouvoir de filtration d'environ 1 µm, et parfait encore le dépoussiérage des fumées, mais comme les fumées la traversant sont déjà fortement dépoussiérées, elle ne se colmate que lentement et peut être remplacée seulement occasionnellement lors d'un arrêt prolongé du four.

La pompe 15, qui a par exemple un débit nominal de 800 I/heure, est préférentiellement du type compresseur à membrane sèche, conditionné, pour résister aux températures encore élevées des fumées qui la traversent. A la sortie de la pompe 15, les fumées sont partagées en deux flux gazeux au moyen du régulateur de débit 17 qui a pour fonction de maintenir constant le débit dans le conduit de liaison 19 et dans l'analyseur 21 par exemple à environ 200 I/h. Le débit excédentaire qui passe dans le conduit d'évacuation 16, varie donc en fonction du degré de colmatage de la ligne de prélèvement qui influe sur le débit effectivement pompé. Le débitmètre a seuil 18 permet de visualiser ce débit excédentaire. Lorsque ce débit, initialement donc à 600 l/h, devient trop faible, par exemple environ 400 I/heure, suite à une diminution du débit réel de la pompe (donc du débit de prélèvement), le débitmètre à seuil 18 fournit un signal électrique, qui, comme on le verra par la suite, commande la purge du circuit de prélèvement et le décolmatage du filtre primaire 11.

Pour éviter une condensation éventuelle de vapeur d'eau, les fumées doivent être à une température supérieure à la température de rosée. Pour maintenir cette température, le coffret 13 est chauffé par une résistance électrique 26, et de même le conduit de liaison 19, entre le coffret 13 et l'analyseur 21 est chauffé par une résistance électrique 27, l'ensemble étant thermostaté à environ 60°C.

Le dispositif comporte également un coffret de pilotage 28 qui regroupe des détendeurs pneumatiques 29, 30, un régulateur 31, des débitmètres 32, 33 et des électrovannes 57, 58 qui permettent d'alimenter manuellement ou automatiquement l'analyseur en air ou en gaz étalon pour le contrôle et l'étalonnage de l'analyseur.

Le coffret comporte encore une vanne 34 reliée à une arrivée d'azote 35, et, par une tuyauterie 36, à la ligne de prélèvement, entre la vanne d'isolement 7 et le filtre primaire 11. Ce circuit est destiné à alimenter en azote la ligne de prélèvement, après l'avoir purgé, pour vérifier son étanchéité. Pour cela, après avoir fermé la vanne d'isolement 7, le dispositif est mis en fonctionnement normal, tout défaut d'étanchéité en amont de la pompe entraînant une aspiration d'air, détectée par l'analyseur 21.

Le coffret 28 est alimenté en air comprimé à travers un filtre déshuileur 37, relié par une conduite 38 au détendeur 29, réglé à une pression de 1,2 bars, dont la sortie est reliée à l'électrovanne 57 par la conduite 39. L'électrovanne 57 est reliée à l'électrovanne à trois voies 58, raccordée à une alimentation 40 en un gaz étalon contenant 0,3 % d'oxygène. L'électrovanne 58 est également reliée au régulateur 31, dont la sortie est connectée par une conduite 41, qui porte le débitmètre 32, à une seconde électrovanne trois voies 56, placée sur le conduit de liaison 19.

Une électrovanne 51 est placée sur la ligne de prélèvement entre le filtre primaire 11 et le condenseur 12. Une électrovanne 59, alimentée en azote sous 7 bars, se raccordant sur cette ligne en amont du condenseur 12. Une électrovanne 54 est placée sur la ligne de prélèvement en aval du condenseur 12, une troisième électrovanne trois voies 55 étant interposée avant le filtre 14, la troisième voie de cette électrovanne étant mise à l'air libre.

Le fonctionnement du dispositif est contrôlé par un automate qui commande les diverses électrovannes et le fonctionnement de la pompe, afin d'assurer un fonctionnement continu sans intervention manuelle pendant une longue durée, par exemple une semaine. Pour cela, il est indispensable de nettoyer fréquemment le filtre primaire 11, le condenseur 12 et les conduits du circuit de prélèvement. Il faut aussi, pour valider la mesure, contrôler une éventuelle dérive de l'analyseur en lui injectant périodiquement des gaz étalons. Ces actions sont gérées par l'automate qui déclenche un cycle de purge et de contrôle à chaque fois que le four est mis hors tension, ou que sa voûte est ouverte, et lorsque le colmatage du circuit de prélèvement est tel que le débit de l'échantillon de fumées prélevé risque de devenir insuffisant pour assurer le faible temps de réponse souhaité, ce qui est signalé par le débitmètre à seuil 18.

La séquence de purge et de contrôle de l'analyseur est indiquée dans le tableau ci-dessous qui indique, pour chaque étape, la position d'ouverture O ou de fermeture F des diverses électrovannes, et l'arrêt ou la marche de la pompe 15. Dans ce tableau, les électrovannes 55 et 56 sont indiquées ouvertes O lorsqu'elles permettent le passage de l'échantillon de fumées, et l'électrovanne 58 est indiquée ouverte lorsqu'elle permet le passage du gaz étalon à 0,3 % d'oxygène.

| Etape | 51 | 52 59 | 53 | 54 | 55 | 56 | Pompe | 57 | 58 |
|---|---|---|---|---|---|---|---|---|---|
| 1 | O | F | F | O | O | O | Marche | F | F |
| 2 | F | O | O | F | F | F | Arrêt | F | O |
| 3 | F | O | F | O | F | F | Arrêt | O | F |
| 4 | O | F | F | O | O | O | Marche | F | F |

L'étape 1 correspond au fonctionnement normal du dispositif, pour le prélèvement et l'analyse des fumées.

Lors de l'étape 2, déclenchée automatiquement par le débitmètre à seuil 18, le filtre primaire 11 est nettoyé par l'azote insufflé à contre courant par la vanne 52, le condenseur est purgé par injection d'azote par la vanne 59 et vidange du pot de récupération 25, et le gaz étalon à 0,3 % d'oxygène est envoyé dans l'analyseur 21, en passant par la vanne 58, le régulateur 31, la vanne 56 et le conduit de liaison 19, pour étalonnage de l'analyseur.

Lors de l'étape 3, le nettoyage du filtre primaire se poursuit, l'azote injecté par la vanne 59 traverse le condenseur et purge la conduite jusqu'à la vanne 55 où il sort à l'air libre ; en même temps, de l'air propre est envoyé dans l'analyseur pour son étalonnage, en passant par le filtre déshuileur 37, le détendeur 29, les vannes 57 et 58, d'où il rejoint l'analyseur.

La quatrième étape est la remise du dispositif en configuration de fonctionnement normal d'analyse.

Au terme de la quatrième étape, l'automate contrôle l'efficacité de la purge en testant l'état du débitmètre à seuil. Si ce débit est insuffisant, le cycle de purge recommence. Si le débit est correct, l'automate envoie un signal à l'opérateur indiquant la validité de la mesure.

Le dispositif et son mode d'utilisation décrits ci-dessus permettent d'obtenir un temps de réponse de l'analyse d'oxygène inférieur à 10 secondes, donc très court, et pouvant être conservé pendant une longue période d'exploitation du four, sans nécessiter d'intervention manuelle. L'autocontrôle de l'analyseur permet par ailleurs de garantir une précision d'analyse de 1 % de la teneur en oxygène mesuré.

L'invention n'est pas limitée au dispositif et procédé décrits ci-dessus à titre d'exemple. En particulier, pour augmenter la fiabilité du système et éviter l'arrêt de l'analyse pendant la période de purge et nettoyage des filtres, on peut utiliser deux filtres primaires et deux sondes de prélèvement, montés en parallèle, et prélever les fumées par l'un des ensembles sonde plus filtre, pendant que l'autre filtre est en cours de nettoyage. L'analyseur peut aussi être un analyseur d'autre type que celui à cellule au zircone, et être choisi pour mesurer la teneur d'autres gaz que l'oxygène.

## Revendications

1. Procédé d'analyse rapide en continu d'un mélange gazeux, selon lequel on fait passer dans un analyseur de gaz (21) un échantillon prélevé dans le dit mélange gazeux, on utilise des moyens de filtration (10), placés en aval d'une sonde de prélèvement (5) et en amont d'une pompe (15), pour épurer le mélange prélevé, on prélève le dit mélange à travers lesdits moyens de filtration, avec un débit élevé supérieur à un débit d'entrée voulu dans l'analyseur, et on dirige le mélange gazeux vers l'analyseur sous ledit débit d'entrée voulu, le débit restant constituant un débit de fuite que l'on évacue sans le faire passer dans l'analyseur, caractérisé en ce qu'on régule le débit d'entrée dans l'analyseur (21), on mesure le débit de fuite évacué, et on procède à un nettoyage des moyens de filtration (10) par un balayage à contre courant des dits moyens de filtration avec un gaz propre, lorsque le débit de fuite mesuré atteint un seuil minimal prédéterminé.

2. Procédé selon la revendication 1, caractérisé en ce que l'on régule le débit d'entrée dans l'analyseur (21) en réglant le débit de fuite.

3. Procédé selon la revendication 1 ou 2, appliqué à l'analyse rapide des fumées de captage d'un four à arc (1), caractérisé en ce qu'on procède au nettoyage des moyens de filtration (10) et à un réétalonnage de l'analyseur (21) seulement quand le four est mis hors tension, ou que sa voûte (2) est ouverte.

4. Dispositif d'analyse rapide en continu d'un mélange gazeux comprenant un analyseur de gaz (21), une ligne de prélèvement comportant une sonde (5) de prélèvement, une pompe (15) pour faire passer dans l'analyseur un échantillon du mélange gazeux prélevé par la sonde, et des moyens de filtration (10), placés en aval de la sonde, la pompe (15) étant placée en aval des moyens de filtration (10), et la sortie de la pompe étant reliée, d'une part, à l'analyseur par un conduit de liaison (19), et, d'autre part, à une évacuation par un conduit d'évacuation (16), caractérisé en ce qu'il comporte un régulateur de débit (17), placé en aval de la pompe (15), pour assurer un débit de mélange gazeux sensiblement constant dans le conduit de liaison (19), des moyens (22, 52) d'injection d'un gaz propre raccordés en aval des moyens de filtration (11), pour faire circuler le dit gaz propre à contre courant dans les moyens de filtration, des moyens (18) de mesure du débit dans le conduit d'évacuation (16), et des moyens de commande pour commander l'injection de gaz propre quand ledit débit dans le conduit d'évacuation (16) atteint un seuil minimal prédéterminé.

5. Dispositif selon la revendication 4, caractérisé en ce que le régulateur de débit (17) est placé sur le conduit d'évacuation (16).

6. Dispositif selon l'une des revendications 4 ou 5, caractérisé en ce que les moyens de filtration (10) comprennent un filtre primaire (11) pour retenir les particules de dimensions supérieures à une dimension prédéterminée et un condenseur (12), placé en aval du filtre primaire.

7. Dispositif selon l'une des revendications 4 à 6, caractérisé en ce qu'il comporte des moyens d'isolement (7) du circuit placés en amont des moyens de filtration (10) pour isoler le circuit de la sonde (5), et des moyens (40, 58, 31) pour injecter dans le circuit un gaz étalon.

## Patentansprüche

1. Verfahren zur kontinuierlichen Schnellanalyse eines Gasgemisches, gemäß dem man eine dem Gasgemisch entnommene Probe in einen Gasanalysator (21) strömen läßt, man Filtermittel (10) verwendet, die stromabwärts von einer Entnahmesonde (5) und stromaufwärts von einer Pumpe (15) angeordnet sind, um das entnommene Gemisch zu reinigen, man das Gemisch mit einer hohen Förderleistung, die größer ist als eine gewünschte Einströmrate in den Analysator, durch diese Filtermittel hindurch entnimmt und man das Gasgemisch mit der gewünschten Einströmrate zum Analysator leitet, wobei der übrige Förderstrom einen Leckstrom bildet, den man abführt, ohne daß er in den Analysator gelangt, dadurch gekennzeichnet, daß man die Einströmrate in den Analysator (21) regelt, man den abgeführten Leckstrom mißt und man eine Reinigung der Filtermittel (10) durch eine Gegenstromspülung der Filtermittel mit einem sauberen Gas vornimmt, wenn der gemessene Leckstrom eine vorbestimmte Mindestschwelle erreicht.

2. Verfahren nach Anspruch 1, dadurch gekennzeichnet, daß man die Einströmrate in den Analysator (21) durch Einstellen des Leckstromes regelt.

3. Verfahren nach Anspruch 1 oder 2, das auf die Schnellanalyse von aufgefangenen Rauchgasen eines Lichtbogenofens (1) angewendet wird, dadurch gekennzeichnet, daß man die Reinigung der Filtermittel (10) und eine erneute Eichung des Analysators (21) nur dann vornimmt, wenn der Ofen ausgeschaltet ist oder sein Gewölbe (2) geöffnet ist.

4. Vorrichtung zur kontinuierlichen Schnellanalyse eines Gasgemisches, umfassend einen Gasanalysator (21) und eine Entnahmeleitung, die eine Entnahmesonde (5), eine Pumpe (15), um eine Probe des von der Sonde entnommenen Gasgemisches in den Analysator zu befördern, und Filtermittel (10) aufweist, die stromabwärts von der Sonde angeordnet sind, wobei die Pumpe (15) stromabwärts von den Filtermitteln (10) angeordnet ist und der Ausgang der Pumpe einerseits durch eine Verbindungsleitung (19) mit dem Analysator und andererseits durch eine Ablaßleitung (16) mit einem Ablaß verbunden ist, dadurch gekennzeichnet, daß sie einen Durchflußmengenregler (17), der stromabwärts von der Pumpe (15) angeordnet ist, um einen im wesentlichen konstanten Förderstrom des Gasgemisches in der Verbindungsleitung (19) zu gewährleisten, Mittel (22, 52) zum Einblasen eines sauberen Gases, die stromabwärts von den Filtermitteln (11) angeschlossen sind, um das saubere Gas im Gegenstrom in die Filtermittel strömen zu lassen, Mittel (18) zum Messen der Durchflußmenge in der Ablaßleitung (16) und Steuermittel aufweist, um das Einblasen von sauberem Gas zu befehlen, wenn die Durchflußmenge in der Ablaßleitung (16) eine vorbestimmte Mindestschwelle erreicht.

5. Vorrichtung nach Anspruch 4, dadurch gekennzeichnet, daß der Durchflußmengenregler (17) auf der Ablaßleitung (16) angeordnet ist.

6. Vorrichtung nach einem der Ansprüche 4 oder 5, dadurch gekennzeichnet, daß die Filtermittel (10) einen Primärfilter (11), um die Teilchen zurückzuhalten, deren Abmessungen größer sind als eine vorbestimmte Abmessung, und einen Kühler (12) umfassen, der stromabwärts von dem Primärfilter angeordnet ist.

7. Vorrichtung nach einem der Ansprüche 4 bis 6, dadurch gekennzeichnet, daß es Leitungstrennmittel (7), die stromaufwärts von den Filtermitteln (10) angeordnet sind, um die Leitung von der Sonde (5) zu trennen, und Mittel (40, 58, 31) aufweist, um in die Leitung ein Eichgas einzublasen.

## Claims

1. Method for the continuous rapid analysis of a gas mixture, in which a sample taken from the said gas mixture is passed through a gas analyser (21), filtration means (10), placed downstream of a sampling probe (5) and upstream of a pump (15), are used to purify the sampled mixture, the said mixture is taken through the said filtration means, with a high flow rate greater than a desired flow rate at entry into the analyser, and the gas mixture is conducted towards the analyser at the said desired entry flow rate, the remaining flow constituting a leakage flow which is exhausted without passing it through the analyser, characterized in that the flow rate at entry into the analyser (21) is regulated, the flow rate of the exhausted leakage flow is measured and a procedure of cleaning the filtration means (10), by backflushing the said filtration means with a clean gas, is carried out when the measured leakage flow rate reaches a predetermined minimum threshold.

2. Method according to Claim 1, characterized in that the flow rate at entry into the analyser (21) is regulated by setting the leakage flow rate.

3. Method according to Claim 1 or 2, applied to the rapid analysis of the flue gases collected from an arc furnace (1), characterized in that the cleaning of the filtration means (10) and a recalibration of the analyser (21) are carried out only when the furnace is switched off or when its roof (2) is open.

4. Device for the continuous rapid analysis of a gas mixture comprising a gas analyser (21), a sampling line which includes a sampling probe (5), a pump (15) for passing a sample of the gas mixture sampled by the probe through the analyser, and filtration means (10) placed downstream of the probe, the pump (15) being placed downstream of the filtration means (10) and the outlet of the pump being connected, on the one hand, to the analyser via a connecting pipe (19) and, on the other hand, to an exhaust via an exhaust pipe (16), characterized in that it includes a flow rate controller (17), placed downstream of the pump (15), in order to provide a substantially constant gas mixture flow rate in the connecting pipe (19), means (22, 52) for injecting a clean gas, these means being connected downstream of the filtration means (11), in order to make the said clean gas flow as a counter current through the filtration means, means (18) for measuring the flow rate in the exhaust pipe (16) and control means for controlling the injection of clean gas when the said flow rate in the exhaust pipe (16) reaches a predetermined minimum threshold.

5. Device according to Claim 4, characterized in that the flow rate controller (17) is placed on the exhaust pipe (16).

6. Device according to either of Claims 4 and 5, characterized in that the filtration means (10) comprise a primary filter (11), in order to retain the particles of sizes greater than a predetermined size, and a condenser (12) placed downstream of the primary filter.

7. Device according to one of Claims 4 to 6, characterized in that it includes means (7) for isolating the circuit, these being placed upstream of the filtration means (10) and intended to isolate the circuit from the probe (5), and means (40, 58, 31) for injecting a standard gas into the circuit.
